# EUROPÄISCHE PATENTSCHRIFT

(11) **EP 0 915 091 B1**
(45) Veröffentlichungstag und Bekanntmachung des Hinweises auf die Patenterteilung: **13.07.2005**
(21) Anmeldenummer: 98120069.4
(22) Anmeldetag: 23.10.1998
(51) Int. Cl.: C07D 307/20

(54) **Verfahren zur Herstellung von fünf- oder sechsgliedrigen cyclischen Ethern, insbesondere Anhydropolyolen**
Process of preparation of five- or sixmembered cyclic ethers, in particular anhydropolyols
Procédé pour la préparation des ethers cycliques à cinque et six chainons, spécialement anhydropolyols

(30) Priorität: 07.11.1997 DE 19749202
(43) Veröffentlichungstag der Anmeldung: 12.05.1999
(73) Patentinhaber: Degussa AG, 40474 Düsseldorf (DE)
(72) Erfinder: Haas, Thomas Dr., 60316 Frankfurt (DE); Burkhardt, Olaf Dr., 63577 Alzenau (DE); Morawietz, Marcus Dr., 63457 Hanau (DE); Vanheertum, Rudolf Dr., 63796 Kahl (DE); Bourrel, Agnes, 75015 Paris (FR)

(56) Entgegenhaltungen:
- EP-A- 0 380 402
- WO-A-89/00162
- DE-A- 3 111 092
- US-A- 3 766 179
- US-A- 4 866 188
- C. MONTASSIER ET AL.: "Polyol conversion into furanic derivatives on bimetallic catalysts; nature of the catalytic sites" JOURNAL OF MOLECULAR CATALYSIS, Bd. 91, 1994, Seiten 119-128, XP002093254

## Beschreibung

Die Erfindung betrifft ein Verfahren zur Herstellung von fünf- oder sechsgliedrigen cyclischen Ethern, insbesondere Anhydropolyolen, durch sauer katalysierte Cyclodehydratation von Polyolen, welche mindestens zwei Hydroxylgruppen in einem eine Ringbildung ermöglichenden Abstand, vorzugsweise also einem Abstand von 4 oder 5 C-Atomen, aufweisen. Die Erfindung richtet sich insbesondere auf die Herstellung von Anhydrotetritolen, Anhydropentitolen und besonders bevorzugt Anhydrohexitolen aus Tetritolen, Pentitolen beziehungsweise Hexitolen.

Die säurekatalysierte Cyclodehydratisierung mehrwertiger Alkohole unter Bildung insbesondere 5-gliedriger cyclischer Ether oder Hydroxyether, nachfolgend als Anhydropolyole bezeichnet, ist lange bekannt. Die Cyclodehydratisierung eines Zuckeralkohols aus der Reihe der Hexitole führt zu einem komplexen Produktgemisch von Anhydro- und Dianhydrohexitolen sowie herstellungsbedingt entstehenden unerwünschten Nebenprodukten, darunter Polymeren - siehe K. Bock et al. in Acta Chemica Scandinavica B 35 (1981) 441-449 und G. Flèche et al. in Starch/Stärke 38 (1986) Nr. 1, 26-30. Anhydropolyole, welche aus nachwachsenden Rohstoffen, wie Zuckern, herstellbar sind, so etwa 2,5-Sorbitan und Isosorbid aus D-Glucose oder Saccharose via Sorbitol, gewinnen zunehmend Interesse bei der Herstellung von Polyesterharzen, Epoxidharzen und Tensiden.

In dem oben zitierten Artikel von G. Flèche wird der Einfluß des Wassergehalts, der Art der Säure und Säurekonzentration auf die Produktzusammensetzung und den Gehalt an Polymeren bei der säurekatalysierten Cyclodehydratation von Sorbitol, das durch Hydrolyse von Stärke und Hydrierung der dabei entstehenden D-Glucose erhalten wird, behandelt. Hiernach wird empfohlen, die Dehydratation möglichst in Abwesenheit von Wasser durchzuführen. Als Katalysatoren werden Mineralsäuren, organische Kationenaustauscher oder Lewis-Säuren eingesetzt. Der Einsatz von Mineralsäuren oder Lewis-Säure als Katalysatoren macht eine Neutralisation und aufwendige Abtrennung des Salzes aus dem Reaktionsgemisch und Entsorgung des Salzes erforderlich. Nachteil aller Ausführungsformen ist der meist hohe Polymergehalt im Reaktionsgemisch. Der Polymergehalt ist insbesondere dann von Nachteil, wenn das Reaktionsgemisch nach Abdestillieren des gebildeten Isosorbids direkt, also ohne weitere aufwendige Reinigungsstufen, einer weiteren Verwertung zugeführt werden soll.

Gemäß DE-OS 31 11 092 läßt sich die Cyclodehydratisierung auch durch gasförmigen Halogenwasserstoff, wie HCl, als Katalysator und zusätzlich gegebenenfalls einer organischen Carbonsäure als Cokatalysator durchführen. Nachteilig ist die sehr hohe Katalysatormenge, sowie ein geringer Anteil von Monoanhydroprodukten. Die Cyclodehydratisierung von Hexitolen läßt sich gemäß WO 89/00162 bei mäßiger Temperatur auch in flüssigem Fluorwasserstoff in Gegenwart einer Carbonsäure durchführen, jedoch ist dieses Verfahren wegen der großen Gefahren von HF für Mensch und Material sehr aufwendig.

Anstelle durch saure Katalysatoren läßt sich die Cyclodehydratation von Polyolen, wie Glucitol, auch durch Bimetall-Katalysatoren, wie Cu-Pt, Cu-Au, Cu-Pd und Cu-Ru, in Gegenwart von Wasserstoff katalysieren - siehe C. Montassier et al., Applied Catalysis A : General 121 (1995), 231-244. Bei dieser Umsetzung kommt es aber mit zunehmendem Umsatz an Glucitol (= Sorbitol) zu Monoanhydroglucitolen und 1,4 : 3,6-Dianhydroglucitol zu einer Abnahme der Dehydratationsselektivität. Die Selektivität kann durch NaCl-Zugabe zwar wieder erhöht werden, jedoch sinkt dabei die Aktivität des Katalysators stark ab. Gemäß EP-B 0 380 402 lassen sich aus Glucitol maximal 71 % Anhydroverbindungen gewinnen (49 % Isosorbid und 22 % isomere Monoanhydroglucitole). Nachteilg sind der hohe Verlust (29 %) an Ausgangsverbindung, die langen Reaktionszeiten und der hohe Katalysatorbedarf beziehungsweise die Notwendigkeit einer Katalysatorregenerierung. Sofern keine langen Reaktionszeiten in Betracht gezogen werden, ist der Umsatz an Sorbitol unvollständig, so daß das cyclodehydratisierte Reaktionsgemisch außer den Monoanhydrohexitolen und Isosorbid noch erhebliche Mengen an Sorbitol enthält. ' Isosorbid läßt sich zwar leicht destillativ aus dem Gemisch abtrennen, der verbleibende Destillationssumpf enthält aber außer den Monoanhydrohexitolen Sorbitol, wobei letzteres Folgeumsetzungen nachteilig beeinflußt oder eine aufwendige Reinigung erforderlich macht.

Aufgabe der Erfindung ist demgemäß ein Verfahren zur Cyclodehydratisierung von Polyolen, insbesondere Zuckeralkoholen, bereitzustellen, das zu einem im wesentlichen quantitativen Umsatz führt und das zu Cyclodehydratisierungsprodukten führt, welche weniger als 1 Gew.-% Polymere enthalten.

Gefunden wurde' ein Verfahren zur Herstellung von fünf- oder sechsgliedrigen cyclischen Ethern, insbesondere Anhydropolyolen, durch Cyclodehydratation von Polyolen mit mindestens 4 C-Atomen und mindestens 2 Hydroxylgruppen, wobei das Polyol in Gegenwart von Wasser und eines sauren Katalysator bei mindestens 100 °C behandelt wird, das dadurch gekennzeichnet ist, daß man die Behandlung in Gegenwart eines säurestabilen Hydrierkatalysators in einer Wasserstoffatmosphäre durchführt.

Im erfindungsgemäßen Verfahren werden Polyole eingesetzt, welche zumindest zwei Hydroxylgruppen in einem eine Cyclodehydratisierung ermöglichenden Abstand enthalten. Üblicherweise sind die Hydroxylgruppen durch vier oder , fünf, vorzugsweise jedoch vier C-Atome voneinander getrennt, so daß bei der Cyclodehydratisierung ein 5-gliedriger oder 6-gliedriger cyclischer Ether gebildet wird. Es ist nicht auszuschließen, daß erfindungsgemäß auch 4-gliedrige cyclische Ether gebildet werden können. Vorzugsweise enthalten die eingesetzten Polyole mehr als zwei Hydroxylgruppen, woraus Anhydropolyole gebildet werden können. Unter dem Begriff "Anhydropolyole" werden hierbei Verbindungen verstanden, welche einen, zwei oder mehrere cyclische Ether mit fünf oder sechs Gliedern, insbesondere fünf Gliedern (= Di- und insbesondere Tetrahydrofuranderivate), und zusätzlich eine oder mehrere, insbesondere zwei bis vier Hydroxylgruppen aufweisen. Die zu dehydratisierenden Polyole enthalten mindestens 4 C-Atome, üblicherweise 4 bis 20 C-Atome, jedoch sind auch Polyole mit mehr als 20 C-Atomen einsetzbar, sofern sie ausreichend wasserlöslich sind. Polyole mit 4 bis 6 C-Atomen und 2 bis 6 Hydroylgruppen sind besonders bevorzugt. Zur letzten Gruppe zählen Zuckeralkohole aus der Reihe der Tetrite, wie Erythritol, Pentite, wie Ribitol, Arabinitol und Xylitol und Hexite, wie Glucitol, Mannitol, Galactitol und Iditol. Einsetzbar sind die Tetrite, Pentite und Hexite in der D- oder L-Form, wie sie üblicherweise in natürlich vorkommenden Rohstoffen oder aus solchen erhältlichen Rohstoffen vorliegen, oder in Form der Racemate oder Gemischen von Konformeren.

Die erfindungsgemäße Cyclodehydratisierung erfolgt in Gegenwart von Wasser. Die Polyole werden üblicherweise in Form einer wäßrigen Lösung, vorzugsweise einer Lösung mit einem Polyolgehalt im Bereich von 10 bis 80 Gew.-%, insbesondere 40 bis 60 Gew.-%, umgesetzt. Einsetzbar sind beispielsweise Lösungen, wie sie bei der Überführung von Stärke in Alditole (= Hexitole = Hexite) durch Hydrolyse mit nachfolgender Hydrierung erhältlich sind.

Die Umsetzung erfolgt bei einer Temperatur von mindestens 100 °C, meistens im Bereich von 120 bis 380 °C. Bevorzugt wird eine Temperatur im Bereich von 120 bis 300 °C, besonders bevorzugt 180 bis 300 °C.

Wie im Stand der Technik sind auch im erfindungsgemäßen Verfahren saure Katalysatoren anwesend. Einsetzbar sind Mineralsäuren, wie H₂SO₄, HCl und H₃PO₄, organische Carbon- und Sulfonsäuren sowie saure Feststoffkatalysatoren, deren Hₒ-Wert der Hammet'schen Säurefunktion kleiner +2, insbesondere kleiner -3 ist. Mineralsäuren werden weniger bevorzugt, weil diese nach der Umsetzung neutralisiert und die Salze aus dem Reaktionsgemisch abgetrennt und entsorgt werden müssen.

Um die Aufarbeitung des Reaktionsgemischs der Cyclodehydratisierung möglichst einfach zu gestalten, wird gemäß einer bevorzugten Ausführungsform eine Carbonsäure, deren Siedepunkt unterhalb desjenigen der abzudestillierenden Mono- oder Dianhydropolyole liegt, insbesondere eine C₁- bis C₁₂-Monocarbonsäure, als Katalysator eingesetzt. Besonders bevorzugt wird eine Carbonsäure aus der Reihe Ameisensäure, Essigsäure und Propionsäure. Derartige Carbonsäuren lassen sich destillativ aus dem Reaktionsgemisch abtrennen und recyclieren.

Bei den säuren Feststoffkatalysatoren mit Hₒ kleiner +2 handelt es sich um Stoffe aus den Reihen: natürliche und synthetische silikatische Stoffe, wie Montmorillonit, Mordenit und saure Zeolithe; an anorganischen Trägerstoffen, wie SiO₂, Al₂O₃ oder TiO₂, fest gebundene Säuren, wie insbesondere Phosphoroxide/-säuren; Oxide, wie gamma-Al₂O₃, TiO₂, ZrO₂, SnO₂, Bi₂O₅, Sb₂O₅, MoO₃, WO₃; Mischoxide, wie SiO₂-Al₂O₃, SiO₂-TiO₂, Al₂O₃-ZnO, SiO₂-ZrO₂, SiO₂-SnO₂, SiO₂-MoO₃, SiO₂-WO₃; Heteropolysäuren, zum Beispiel Polywolframatosilikate und Polywolframatophosphate; Metallsalze, wie AlPO₄, FePO₄, Zn₃(PO₄)₂, Mg₃(PO₄)₂, Ti₃(PO₄)₄, Zr₃(PO₄)₄; Kationenaustauscher, wie Sulfonatgruppen enthaltende Austauscher auf der Basis von Polystyrol, polymeren perfluorierten Harzen oder vorzugsweise Organopolysiloxanen (Deloxan® der Degussa AG). Besonders bevorzugte saure Feststoffkatalysatoren für die erfindungsgemäße Etherbildung und -spaltung sind Zeolithe vom Typ H-Y und H-ZSM 5.

Die Einsatzmenge von im Reaktionsgemisch löslichen sauren Katalysatoren liegt im allgemeinen im Bereich von 0,1 bis 20 Gew.-%, insbesondere 0,5 und 10 Gew.-%, jeweils bezogen auf das Polyol. Die Einsatzmenge fester saurer Katalysatoren richtet sich sowohl nach deren Aktivität und der gewählten Reaktionstemperatur, liegt im allgemeinen aber im Bereich der löslichen Katalysatoren. Die optimale Einsatzmenge läßt sich durch orientierende Versuche einfach ermitteln.

Erfindungswesentliches Merkmal ist, daß zusätzlich zum sauren Katalysator ein üblicher Hydrierkatalysator anwesend ist und die Cyclodehydratisierung in einer Wasserstoffatmosphäre durchgeführt wird. Der Wasserstoffpartialdruck liegt im allgemeinen im Bereich von mindestens 0,1 bis 20 MPa, vorzugsweise im Bereich von 1 bis 15 MPa und insbesondere 3 bis 10 MPa.

Als Hydrierkatalysatoren können zwar homogene und heterogene Katalysatoren eingesetzt werden, jedoch werden heterogene Katalysatoren bevorzugt, weil damit eine einfache Abtrennung des Katalysators vom Reaktionsgemisch, etwa durch Filtration, möglich ist. Übliche Hydrierkatalysatoren enthalten als wirksame Komponente ein Edelmetall aus der Reihe Ru, Rh, Pd und Pt, oder ein Übergangsmetall aus der Reihe Cu, Cr, Co, Ni, Fe, darunter insbesondere Raney-Katalysatoren und Chromit-Katalysatoren; einsetzbar sind auch Bimetall-Katalysatoren aus einem Übergangsmetall und Edelmetall. Die Verwendung eines ein oder mehrere Übergangsmetalle enthaltenden Hydrierkatalysators ist nur dann zweckmäßig, wenn der Katalysator unter den Reaktionsbedingungen eine ausreichende Säurestabilität aufweist.

Bevorzugte Hydrierkatalysatoren für das erfindungsgemäße Verfahren sind Edelmetallkatalysatoren in metallischer Form, wie sogenannte Mohre von Ru, Rh und insbesondere Pd und Pt, oder an einen Träger gebundenen Form. Geeignete Trägermaterialien für Ru, Rh, Pd und Pt sind Aktivkohle, Aluminiumoxid und andere Metalloxide sowie Silikate. Die Edelmetallmenge trägergebundener Edelmetallkatalysatoren liegt meist im Bereich von 0,0001 bis 10 Gew.-%, bei Pd und Ru vorzugsweise im Bereich von 0,01 bis 0,1 Gew.-%. Die Einsatzmenge an Edelmetallkatalysatoren, welche von der Aktivität des Katalysators, der Reaktionstemperatur und dem H₂-Druck abhängt, wird der Fachmann durch orientierende Versuche ermitteln. Im allgemeinen liegt die Einsatzmenge im Bereich von 0,01 bis 10 Gew.-%, insbesondere 0,5 bis 5 Gew.-%, bezogen auf das Polyol. Edelmetallkatalysatoren in Form eines Mohrs oder trägergebunden lassen sich leicht recyclieren, sie weisen eine höhere Standzeit auf als Bimetall-Katalysatoren auf der Basis eines Edelmetalls und, eines Übergangsmetalls, wie sie im vorbekannten Verfahren zur Cyclodehydratation in Abwesenheit eines sauren Katalysators eingesetzt wurden.

Das Verfahren kann diskontinuierlich oder kontinuierlich betrieben werden. Hierbei können das Polyol und Wasser vor dem Reaktor gemischt oder parallel dem Reaktor zugeführt werden. Bei Verwendung einer im Reaktionsgemisch löslichen Säure als Katalysator wird dies dem Reaktionspartner, dem Wasser oder Gemisch aus beiden zugesetzt oder separat in den Reaktor eingeführt. Ein fester Hydrierkatalysator kann als Suspensionskatalysator oder als Festbett eingesetzt werden. Bei Verwendung eines festen sauren Katalysators kann dieser analog zum Hydrierkatalysator als Suspension oder als Festbett zur Anwendung gelangen. Es ist auch möglich, einen sowohl saure als auch hydrierwirksame Funktionen enthaltenden Katalysator, beispielsweise einen teilweise mit einem Edelmetall beladenen Zeolithen, einzusetzen. Die optimale Reaktionszeit kann der Fachmann durch orientierende Versuche leicht ermitteln.

Das Reaktionsgemisch der Cyclodehydratisierung läßt sich in einfacher Weise aufarbeiten. Diese Aufarbeitung kann die Filtration eines festen sauren Katalysators und eines heterogenen Hydrierkatalysators umfassen. Bei Verwendung eines destillierbaren.sauren Katalysators und heterogenen Hydrierkatalysators umfaßt die Aufarbeitung die Filtration der Hydrierkatalysatoren und destillative Abtrennung des sauren Katalysators. Das nach Abtrennung der Katalysatoren verbleibende Reaktionsgemisch wird destillativ und/oder extraktiv, vorzugsweise destillativ, aufgearbeitet. Bei Bedarf kann die Aufarbeitung auch eine Kristallisation umfassen. Im Fall der Cyclodehydratation von Hexitolen, werden entstandene Dianhydrohexitole meistens destillativ abgetrennt und es verbleibt im Destillationssumpf ein Gemisch von Monoanhydrohexitolen.

Es ist ein überraschender und damit wesentlicher Vorteil des erfindungsgemäßen Verfahrens, daß Polymere praktisch nicht gebildet werden - die Bildungsrate liegt unter 1 Mol-%, bezogen auf umgesetztes Polyol. Damit läßt sich das von Katalysatoren und Wasser befreite Reaktionsgemisch unmittelbar oder nach destillativer Abtrennung von destillierbaren Dianhydropolyolen weiterverwerten. Monoanhydropolyole, insbesondere solche aus Hexitolen, sind damit wertvolle Rohstoffe für verschiedene Einsatzgebiete, wo bisher der Gehalt an Polymeren störend war. Durch die bevorzugte Verwendung einer niederen Carbonsäure und eines heterogenen Hydrierkatalysators ist es möglich, nicht nur den Hydrierkatalysator, sondern auch die Carbonsäure nach ihrer destillativen Abtrennung wieder zu rezyklieren. Bei Verwendung von Mineralsäuren waren bisher aufwendige Maßnahmen erforderlich, das durch Neutralisation des sauren Katalysators entstandene Salz aus dem Anhydropolyol-Reaktionsgemisch abzutrennen. Schließlich ist es durch die erfindungsgemäße Kombination eines sauren Katalysators und eines Hydrierkatalysators möglich, Edelmetallkatalysatoren mit hoher Stabilität einzusetzen, was sich vorteilhaft auf die Rezyklierbarkeit und damit die Verfahrenskosten auswirkt.

Bei der Cyclodehydratisierung von Hexitolen lassen sich nach Abdestillation von Dianhydrohexitolen Anhydrohexitolgemische gewinnen, welche eine in der Tabelle angegebene typische Zusammensetzungen aufweisen, wobei die konkrete Zusammensetzung von den gewählten Reaktionsbedingungen abhängt.

**Tabelle:**

| Anhydropolyolgemische aus Hexitolen nach Abdestillation von Dianhydrohexitolen | | | |
|---|---|---|---|
| Polyol (Substrat) | D-Sorbitol (=D-Glucitol) | D-Mannitol | Dulcitol (Galacticol) |
| Zusammensetzung | (%) | (%) | (%) |
| 2,5-Anhydro-D-mannitol | 5 - 50 | | 1 - 5 *) |
| 2,5-Anhydro-L-iditol | 10 - 50 | | 1 - 5 *) |
| 1,4-Anhydro-D-sorbitol | 10 - 70 | | |
| 1,4-Anhydro-DL-galacticol | | | 65 - 95 |
| 1,4-Anhydro-D-mannitol | 0 - 10 | 5 - 20 | 1 - 5 *) |
| 2,5-Anhydro-D-sorbitol | | 50 - 90 | |
| 1,5-Anhydro-D-mannitol | | 5 - 20 | |
| andere Polyole | 1 - 15 | 1 - 10 | 1 - 20 |

| | | | |
|---|---|---|---|
| *) DL-Form des Anhydroprodukts | | | |

Diese Stoffgemische lassen sich zur Herstellung von Tensiden sowie als Komponente in Polykondensations- und Polyadditionsharzen einsetzen. Hierbei ist festzustellen, daß ein erhöhter Anteil an 2,5-Anhydrohexitolen gegenüber 1,4-Anhydrohexitolen deutliche Vorteile in der Weiterverarbeitung besitzt: Aus den 1,4-Anhydrohexitolen können bei ungünstigen Verarbeitungsbedingungen durch eine weitere Kondensation Dianhydroverbindungen entstehen, wogegen bei den 2,5-Anhydrohexitolen diese Reaktion nicht möglich ist, so daß 4 Hydroxyfunktionen für die Weiterverarbeitung zugänglich bleiben.

### Beispiel 1

In einem 20 l-Autoklaven wurden 8 kg D-Sorbitol als 50 Gew.-%ige Lösung in Wasser, 5 Gew.-% Propionsäure, bezogen auf Sorbitol, und 1 Gew.-% Pd/C-Katalysator mit einem Pd-Gehalt von 3 Gew.-%, bezogen auf Sorbitol, vorgelegt. Die Reaktionsmischung wurde auf 270 °C erwärmt und 2 h bei 60 bar H₂-Druck gerührt. Nach dem Abkühlen wurde der Katalysator abfiltriert und das Wasser-Propionsäure-Gemisch abdestilliert. Nach gaschromatographischer Analyse betrug die Ausbeute, bezogen auf D-Sorbitol, 38 % Isosorbid und 58 % Anhydrohexitole (= Tetrole) und weniger als 1 % Polymere. Der Umsatz an D-Sorbitol war praktisch quantitativ.

Unter Hochvakuum (< 10 Pa) wurden bei 130 °C 2,5 kg Isosorbid (= 1,4:3,6-Dianhydro-D-sorbitol) abdestilliert. Der zurückbleibende Sumpf (3,9 kg) enthielt 20 % 2,5-Anhydro-D-mannitol, 31 % 2,5-Anhydro-L-iditol, 34 % 1,4-Anhydro-D-sorbitol und 6 % 1,4-Anhydro-D-mannitol, 5 % Isosorbid und etwa 3 % andere monomere Polyole, jedoch praktisch keine Polymere (< 1 %).

Die Analysen sämtlicher Beispiele und Vergleichsbeispiele erfolgten mittels GC-Analytik der silyierten Polyole auf einer Kapillarsäure (DB-5) bei 280 °C. Die Detektion erfolgt in einem Flammenionisationsdetektor bei 250 °C mit Helium als Trägergas. Nach Retentionszeiten zwischen 15 bis 20 min konnten die Produkte eluiert und identifiziert werden.

### Beispiel 2

Beispiel 1 wurde mit dem Unterschied wiederholt, daß die Cyclodehydratation 8 Stunden bei 240 °C erfolgte. Nach GC-Analyse war der Umsatz größer 99 %; die Ausbeuten (bezogen auf Sorbitol) betrugen 20 % Isosorbid, 65 % Monoanhydrohexitole (= Tetrole) und weniger als 1 % Polymere.

Das von Isosorbid weitgehend befreite Reaktionsgemisch enthielt 14 % 2,5-Anhydro-D-mannitol, 21 % 2,5-Anhydro-L-iditol, 44 % 1,4-Anhydro-D-sorbitol, 4 % 1,4-Anhydro-D-mannitol und etwa 16 % andere Polyole, jedoch weniger als 1 % Polymere.

### Beispiel 3

Es wurde gemäß Beispiel 1 D-Mannitol anstelle von Sorbitol eingesetzt, wodurch eine stereochemisch veränderte Produktzusammensetzung erhalten wurde. Als Hauptkomponenten bildeten sich (bezogen auf D-Mannitol): 48 % 2,5-Anhydro-D-sorbitol und 23 % Isomännid .(= 1,4:3,6-Dianhydro-D-mannitol), 10 % 1,4-Anhydro-D-mannitol und 10 % 1,5-Anhydro-D-mannitol. Das Gemisch enthielt weniger als 1 % D-Mannitol und weniger als 1 % Polymere.

### Beispiel 4

D-Sorbitol wurde analog Beispiel 1 cylcodehydratisiert, wobei jedoch anstelle Propionsäure ein saurer Zeolith (Typ Y-Zeolith) in einer Menge von 1 Gew.-%, bezogen auf D-Sorbitol eingesetzt wurde, die Reaktionstemperatur 270 °C und die Reaktionsdauer 4 Stunden betrug. Der Umsatz an D-Sorbitol war praktisch quantitativ (größer 99 %). Die Ausbeuten (bezogen auf Sorbitol) betrugen 46 % Isosorbid, 45 % Anhydrohexitole, etwa 8 % andere niedermolekulare Polyole und weniger als 1 % Polymere.

### Beispiel 5

D-Sorbitol wurde analog Beispiel 1 dehydratisiert, wobei jedoch anstelle des Hydrierkatalysators Pd/C ein Ru/C-Katalysator mit einem Gehalt von 5 % Ru in einer Menge von 0,1 Gew.-%, bezogen auf Sorbitol, eingesetzt wurde. Der Umsatz betrug 96 %; die Ausbeuten, bezogen auf Sorbitol, betrugen 25 % Isosorbid, 55 % Anhydrohexitole, etwa 19 % andere niedermolekulare Polyole und weniger als 1 % Polymere.

### Beispiel 6

Analog Beispiel 5 wurde cyclodehydratisiert, jedoch wurde anstelle D-Sorbitol Saccharose als Substrat eingesetzt, ferner wurde 8 h bei 150 °C und anschließend 4 h bei 270 °C umgesetzt. Bei dieser Umsetzung erfolgten die Hydrierung der Saccharose zu D-Sorbitol und D-Mannitol in situ mit der Cyclodehydratisierung. Der Umsatz an Saccharose betrug 95 %. Das Reaktionsgemisch bestand aus 29 % Isosorbid (= 1,4:3,6-Dianhydro-D-sorbitol), 7 % Isomannid (= 1,4:3,6-Dianhydro-D-mannitol), 12 % 2,5-Anhydro-D-sorbitol, 13 % 2,5-Anhydro-D-mannitol, 14 % 2,5-Anhydro-L-iditol, 7 % 1,4-Anhydro-D-sorbitol, 5 % 1,4-Anhydro-D-mannitol und 2 % 1, 5-Anhydro-D-mannitol und 11 % andere niedermolekulare Polyole. Polymere würden praktisch nicht gebildet.

### Beispiel 7

Als Substrat wurde 1,4-Butandiol eingesetzt. Die Umsetzung erfolgte ansonsten analog Beispiel 1. Der Umsatz an 1,4-Butandiol betrug 76 %, die Ausbeute an Tetrahydrofuran betrug 52 %.

### Vergleichsbeispiel 1 (ohne sauren Katalysator)

Beispiel 2 wurde mit dem einzigen Unterschied wiederholt, daß kein saurer Katalysator eingesetzt wurde. Nach 8 h bei 240 °C betrug der Umsatz an D-Sorbitol 92 %. Die Ausbeuten, bezogen auf eingesetztes D-Sorbitol, betrugen 70 % Monoanhydrohexitole, 20 % Isosorbid, etwa 9 % andere niedermolekulare Polyole und weniger als 1 % Polymere.

Das Beispiel zeigt, daß in Abwesenheit einer Säure nur ein ungenügender Umsatz an D-Sorbitol erzielt wird.

### Vergleichsbeispiel 2 (ohne Hydrierkatalysator und H₂)

Beispiel 2 wurde mit dem einzigen Unterschied wiederholt, daß kein Hydrierkatalysator zugesetzt wurde. Bei fast quantitativem Umsatz an Sorbitol wurden 8 % desselben in weiß-gelbe Polymere überführt. Die Ausbeuten, bezogen auf eingesetztes D-Sorbitol, betrugen ferner 29 % Isosorbid, 54 % Anhydrohexitole, 9 % andere niedermolekulare Polyole.

### Vergleichsbeispiel 3 bis 6

In Abwesenheit eines sauren Katalysators wurde Sorbit in Form einer 20 Gew.-%igen wäßrigen Lösung mit den in der Tabelle angegebenen Hydrierkatalysatoren bei 240 °C und einem H₂-Druck von 13 MPa 8 h behandelt. Der Umsatz an Sorbit sowie die auf umgesetztes Sorbit bezogene Ausbeute an Isosorbid und Monoanhydropolyolen sind der Tabelle zu entnehmen. Polymere entstanden jeweils in einer Menge von weniger als 1 %. Als Hauptprodukte entstanden Polyole als C₂ bis C₄ Fragmente.

| Nr. | Katalysator | Sorbit Umsatz (%) | Monoanhydrohexitole (= Tetrole) (%) | Isosorbid (%) |
|---|---|---|---|---|
| VB 3 | Raney-Cu | 95 | 4 | < 1 |
| VB 4 | Co-Cu-Mn | 95 | < 1 | < 1 |
| VB 5 | Raney-Ni | 60 | 10 | 2 |
| VB 6 | Cr-Ni | 90 | < 1 | < 1 |

Diese Versuche zeigen, daß die untersuchten Katalysatoren für die Cyclodehydratisierung wenig geeignet sind.

## Patentansprüche

1. Verfahren zur Herstellung von fünf- oder sechsgliedrigen cyclischen Ethern, insbesondere Anhydropolyolen, durch Cyclodehydratation von Polyolen mit mindestens 4 C-Atomen und mindestens 2 Hydroxylgruppen, wobei das Polyol in Gegenwart von Wasser und einem sauren Katalysator bei mindestens 100 °C in wässriger Lösung behandelt wird,
**dadurch gekennzeichnet,**
**daß** man die Behandlung in Gegenwart eines säurestabilen Hydrierkatalysator, enthaltend ein Edelmetall aus der Reihe Ru, Rh, Pd und Pt und/oder ein Übergangsmetall aus der Reihe Cu, Cr, Co, Ni und Fe, in einer Wasserstoffatmosphäre durchführt.

2. Verfahren nach Anspruch 1,
**dadurch gekennzeichnet,**
**daß** man die Cyclodehydratisierung bei einer Temperatur im Bereich von 120 bis 380 °C und einem Wasserstoffdruck im Bereich von 1 bis 20 MPa durchführt.

3. Verfahren nach Anspruch 2,
**dadurch gekennzeichnet,**
**daß** man die Cyclodehydratisierung bei einer Temperatur im Bereich von 180 bis 280 °C und einem H₂-Druck im Bereich,'von 3 bis 10 MPa durchführt.

4. Verfahren nach einem der Ansprüche 1 bis 3,
**dadurch gekennzeichnet,**
**daß** man als Hydrierkatalysator einen ein oder mehrere Edelmetalle aus der Reihe Ruthenium, Rhodium, Palladium und Platin in elementarer Form oder als Edelmetallverbindung enthaltenden Katalysator einsetzt.

5. Verfahren nach einem der Ansprüche 1 bis 4,
**dadurch gekennzeichnet,**
**daß** man den Hydrierkatalysator in einer Menge von 0,001 bis 10 Gew.-%, insbesondere 0,01 bis 1 Gew.-%, bezogen auf das Polyol, einsetzt.

6. Verfahren nach einem der Ansprüche 1 bis 5,
**dadurch gekennzeichnet,**
**daß** man als sauren Katalysator eine aliphatische Carbonsäure mit 1 bis 10 C-Atomen, insbesondere eine Monocarbonsäure aus der Reihe Ameisensäure, Essigsäure und Propionsäure, verwendet.

7. Verfahren nach einem der Ansprüche 1 bis 6,
**dadurch gekennzeichnet,**
**daß** man den sauren Katalysator in einer Menge von 0,1 bis 20 Gew.-%, insbesondere 0,5 bis 10 Gew.-%, jeweils bezogen auf das Polyol, einsetzt.

8. Verfahren nach einem der Ansprüche 1 bis 7,
**dadurch gekennzeichnet,**
**daß** man ein Polyol aus der Reihe der Tetritole, Pentitole und Hexitole cyclodehydratisiert.

9. Verfahren nach einem der Ansprüche 1 bis 8,
**dadurch gekennzeichnet,**
**daß** man einen saure und hydrierwirksame Funktionen enthaltenden Katalysator auf der Basis eines mit einem Edelmetall aus der Reihe Pd, Pt, Ru und Rh beladenen Zeoliths mit einem Hₒ-Wert von kleiner +2, insbesondere kleiner -3, verwendet.

10. Anhydrohexitolgemisch, erhältlich durch Cyclodehydratisierung von Sorbitol gemäß einem der Ansprüche 1 bis 9 mit nachfolgender Abtrennung von Isosorbid (1,4 : 3,6-Dianhydrosorbitol) aus dem Reaktionsgemisch, bestehend aus 5 bis 50 % 2,5-Anhydromannitol, 10 bis 50 % 2,5-Anhydroiditol, 10 bis 70 % 1,4-Anhydrosorbitol, 0 bis 10 % 1,5-Anhydromannitol und 1 bis 15 % anderen Polyolen einschließlich Anhydropolyolen.

11. Anhydrohexitolgemisch, erhältlich durch Cyclodehydratisierung von Mannitol gemäß einem der Ansprüche 1 bis 9 mit nachfolgender Abtrennung von 1,4 : 3,6-Dianhydromannitol aus dem Reaktionsgemisch, bestehend aus 50 bis 90 % 2,5-Anhydrosorbitol, 5 bis 20 % 1,4-Anhydromannitol, 5 bis 20 % 1,5-Anhydromannitol und 1 bis 10 % anderen Polyolen einschließlich Anhydropolyolen.

12. Anhydrohexitolgemisch, erhältlich durch Cyclodehydratisierung von Dulcitol gemäß einem der Ansprüche 1 bis 9 mit nachfolgender Abtrennung von Dianhydrodulcitol, bestehend aus 65 bis 95 % 1,4-Anhydrosorbitol, 1 bis 5 % 2,5-Anhydromannitol, 1 bis 5 % 1,5-Anhydromannitol, 1 bis 5 % 2,5-Anhydroiditol und 1 bis 20 % weiteren Polyolen einschließlich Anhydropolyolen.

## Claims

1. Process for the production of five-membered or six-membered cyclic ethers, in particular anhydropolyols, by cyclodehydration of polyols having at least 4 C atoms and at least 2 hydroxyl groups, the polyol being treated in the presence of water and an acidic catalyst at a temperature of at least 100 °C in aqueous solution,
**characterised in that**
the treatment is carried out in the presence of an acid-stable hydrogenating catalyst, containing a noble metal from the series comprising Ru, Rh, Pd and Pt and/or containing a transition metal from the series comprising Cu, Cr, Co, Ni and Fe, in a hydrogen atmosphere.

2. Process according to Claim 1,
**characterised in that**
the cyclodehydrogenation is carried out at a temperature in the range from 120 °C to 380 °C and at a hydrogen pressure in the range from 1 MPa to 20 MPa.

3. Process according to Claim 2,
**characterised in that**
the cyclodehydrogenation is carried out at a temperature in the range from 180 °C to 280 °C and at a H₂ pressure in the range from 3 MPa to 10 MPa.

4. Process according to one of Claims 1 to 3,
**characterised in that**
by way of hydrogenating catalyst use is made of a catalyst containing one or more noble metals from the series comprising ruthenium, rhodium, palladium and platinum in elemental form or in the form of a noble-metal compound.

5. Process according to one of Claims 1 to 4,
**characterised in that**
the hydrogenating catalyst is employed in an amount from 0.001 wt.% to 10 wt.%, in particular 0.01 wt.% to 1 wt.%, relative to the polyol.

6. Process according to one of Claims 1 to 5,
**characterised in that**
by way of acidic catalyst use is made of an aliphatic carboxylic acid having 1 to 10 C atoms, in particular a monocarboxylic acid from the series comprising formic acid, acetic acid and propionic acid.

7. Process according to one of Claims 1 to 6,
**characterised in that**
the acidic catalyst is employed in an amount from 0.1 wt.% to 20 wt.%, in particular 0.5 wt.% to 10 wt.%, in each case relative to the polyol.

8. Process according to one of Claims 1 to 7,
**characterised in that**
a polyol from the family comprising the tetritols, pentitols and hexitols is cyclodehydrogenated.

9. Process according to one of Claims 1 to 8,
**characterised in that**
use is made of a catalyst containing acidic and hydrogenation-active functions and based on a zeolite charged with a noble metal from the series comprising Pd, Pt, Ru and Rh and having an Hₒ value less than +2, in particular less than -3.

10. Anhydrohexitol mixture, obtainable by cyclodehydrogenation of sorbitol in accordance with one of Claims 1 to 9 with subsequent separation of isosorbide (1,4 : 3,6-dianhydrosorbitol) from the reaction mixture, consisting of 5 % to 50% 2,5-anhydromannitol, 10% to 50% 2,5-anhydroiditol, 10% to 70% 1,4-anhydrosorbitol, 0% to 10% 1,5-anhydromannitol and 1 % to 15% other polyols including anhydropolyols.

11. Anhydrohexitol mixture, obtainable by cyclodehydrogenation of mannitol in accordance with one of Claims 1 to 9 with subsequent separation of 1,4 : 3,6-dianhydromannitol from the reaction mixture, consisting of 50% to 90% 2,5-anhydrosorbitol, 5% to 20 % 1,4-anhydromannitol, 5% to 20 % 1,5-anhydromannitol and 1% to 10% other polyols including anhydropolyols.

12. Anhydrohexitol mixture, obtainable by cyclodehydrogenation of dulcitol in accordance with one of Claims 1 to 9 with subsequent separation of dianhydrodulcitol, consisting of 65 % to 95% 1,4-anhydrosorbitol, 1% to 5% 2,5-anhydromannitol, 1% to 5 % 1,5-anhydromannitol, 1% to 5% 2,5-anhydroiditol and 1% to 20% additional polyols including anhydropolyols.

## Revendications

1. Procédé de production d'éthers cycliques à 5 ou 6 chaînons, en particulier d'anhydropolyols, par cyclodéshydratation de polyols, avec au moins 4 atomes de carbone et au moins deux groupes hydroxyle, le polyol étant traité en présence d'eau et d'un catalyseur acide à au moins 100°C en solution aqueuse,
**caractérisé en ce qu'**
on effectue le traitement en présence d'un catalyseur d'hydrogénation stable vis-à-vis des acides, contenant un métal noble de la série Ru, Rh, Pd et Pt et/ou un métal de transition de la série Cu, Cr, Co, Ni et Fe, dans une atmosphère d'hydrogène.

2. Procédé selon la revendication 1,
**caractérisé en ce qu'**
on conduit la cyclodéshydratation à une température allant de 120 à 380°C et sous une pression d'hydrogène allant de 1 à 20 MPa.

3. Procédé selon la revendication,
**caractérisé en ce qu'**
on conduit la cyclodéshydratation à une température allant de 180 à 280°C et sous une pression d'hydrogène allant de 3 à 10 MPa.

4. Procédé selon l'une des revendications 1 à 3,
**caractérisé en ce qu'**
on utilise comme catalyseur d'hydrogénation un ou plusieurs métaux nobles de la série du ruthénium, du rhodium, du palladium et du platine, sous forme élémentaire ou sous forme de catalyseur contenant un composé de métal noble.

5. Procédé selon l'une des revendications 1 à 4,
**caractérisé en ce qu'**
on utilise le catalyseur d'hydrogénation en une quantité de 0,01 à 10 % en poids, en particulier de 0,01 à 1 % en poids, par rapport au polyol.

6. Procédé selon l'une des revendications 1 à 5,
**caractérisé en ce qu'**
on utilise comme catalyseur acide un acide carboxylique aliphatique comportant de 1 à 10 atomes de carbone, en particulier un acide mono-carboxylique de la série de l'acide formique, de l'acide acétique et de l'acide propionique.

7. Procédé selon l'une des revendications 1 à 6,
**caractérisé en ce qu'**
on utilise le catalyseur acide en une quantité de 0,1 à 20 % en poids, en particulier de 0,5 à 10 % en poids, toujours par rapport au polyol.

8. Procédé selon l'une des revendications 1 à 7,
**caractérisé en ce qu'**
on cyclodéshydrate un polyol de la série des tétritols, des pentitols et des hexitols.

9. Procédé selon l'une des revendications 1 à 8,
**caractérisé en ce qu'**
on utilise un catalyseur contenant des fonctions acides et actif pour l'hydrogénation, à base d'une zéolithe chargée avec un métal noble de la série Pd, Pt, Ru et Rh, ave une valeur d'H₀ inférieure à +2, en particulier inférieure à -3.

10. Mélange d'anhydrohexitols, que l'on peut obtenir par cyclodéshydratation de sorbitol selon l'une des revendications 1 à 9, puis par séparation d'isosorbide (1,4: 3,6-dianhydrosorbitol) du mélange réactionnel, constitué de 5 à 50 % de 2,5-anhydromannitol, 10 à 50 % de 2,5-anhydroiditol, 10 à 70 % de 1,4-ahydrosorbitol, 0 à 10 % de 1,5-nhydromannitol et 1 à 15 % d'autres polyols y compris les anhydropolyols.

11. Mélange d'anhydrohexitols, que l'on peut obtenir par cyclodéshydratation de mannitol selon l'une des revendications 1 à 9, puis par séparation du 1,4: 3,6-dianhydromannitol du mélange réactionnel, constitué de 50 à 90 % de 2,5-anhydrosorbitol, 5 à 20 % de 1,4-anhydromannitol, 5 à 20 % de 1,5-anhydromannitol et 1 à 10 % d'autres polyols y compris les anhydropolyols.

12. Mélange d'anhydrohexitols, que l'on peut obtenir par cyclodéshydratation de dulcitol selon l'une des revendications 1 à 9, puis par séparation du 1,4 : 3,6-dianhydrodulcitol du mélange réactionnel, constitué de 65 à 95 % de 1,4-anhydrosorbitol, 1 à 5 % de 2,5-anhydromannitol, 1 à 5 % de 1,5-anhydromannitol, 1 à 5 % de 2,5-anhydroiditol et 1 à 20 % d'autres polyols y compris les anhydropolyols.
